(19) Europäisches Patentamt
European Patent Office
Office européen des brevets

(11) **EP 4 553 943 A1**

(12) **EUROPEAN PATENT APPLICATION**
published in accordance with Art. 153(4) EPC


(43) Date of publication:
**14.05.2025 Bulletin 2025/20**

(21) Application number: **22950540.9**

(22) Date of filing: **12.07.2022**

(51) International Patent Classification (IPC):
***H01M 10/0567*** *(2010.01)* ***H01M 10/0525*** *(2010.01)*

(52) Cooperative Patent Classification (CPC):
**H01M 10/0525; H01M 10/0567**

(86) International application number:
**PCT/CN2022/105202**

(87) International publication number:
**WO 2024/011409 (18.01.2024 Gazette 2024/03)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**



(71) Applicant: **Ningde Amperex Technology Limited**
**Ningde, Fujian 352100 (CN)**

(72) Inventors:
- **WANG, Rui**
  **Ningde, Fujian 352100 (CN)**
- **WANG, Xiang**
  **Ningde, Fujian 352100 (CN)**
- **CUI, Hui**
  **Ningde, Fujian 352100 (CN)**
- **TANG, Chao**
  **Ningde, Fujian 352100 (CN)**

(74) Representative: **Icosa**
**83 avenue Denfert-Rochereau**
**75014 Paris (FR)**


(54) **ELECTROCHEMICAL DEVICE AND ELECTRONIC DEVICE COMPRISING ELECTROCHEMICAL DEVICE**


(57) This application provides an electrochemical device and an electronic device including the same. The electrochemical device includes a positive electrode plate, a separator, and a negative electrode plate, where the positive electrode plate includes a positive electrode active material layer, and the negative electrode plate includes a negative electrode active material layer. Observed along a thickness direction of the electrochemical device, in a width direction of the negative electrode plate, the negative electrode active material layer includes two protruding regions not overlapping with the positive electrode active material layer on two sides, and width M mm of the protruding region is 0.5 mm to 2 mm. The electrochemical device further includes an electrolyte, the electrolyte includes a polynitrile compound, and the polynitrile compound includes a compound represented by formula I-A. Based on a mass of the electrolyte, a mass percentage N% of the polynitrile compound is 0.01% to 8%. The width M mm of the protruding region and the mass percentage N% of the polynitrile compound satisfy $0.01 \leq M \times N < 10$.


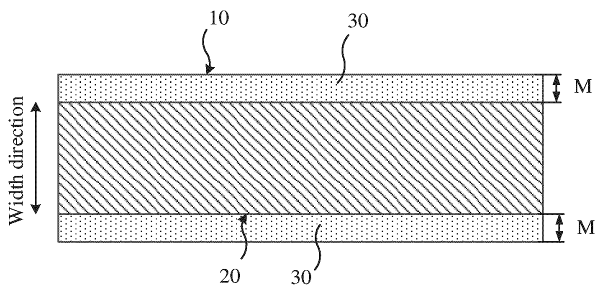


FIG. 1

EP 4 553 943 A1

## Description

### TECHNICAL FIELD

This application relates to the electrochemical field, and specifically, to an electrochemical device and an electronic device including the same.

### BACKGROUND

Lithium-ion batteries, due to their advantages such as high energy density, long cycle life, and no memory effect, are widely used in fields such as smart phones, wearable devices, consumer-grade drones, and electric vehicles. With the wide use of lithium-ion batteries in these fields, the market imposes increasingly high requirements for the energy density of lithium-ion batteries. As the energy density increases, the voltage of lithium-ion batteries also rises.

However, the increase in the voltage of lithium-ion batteries exacerbates the damage to the positive electrode active material and deteriorates the chemical stability of the electrolyte, in turn affecting the floating charge performance of lithium-ion batteries.

### SUMMARY

This application provides an electrochemical device and an electronic device including the same, so as to improve floating charge performance of the electrochemical device.

It should be noted that in the invention content of this application, an example in which a lithium-ion battery is used as an electrochemical device is used to illustrate this application. However, the electrochemical device of this application is not limited to the lithium-ion battery. Specific technical solutions are as follows:

A first aspect of this application provides an electrochemical device including a positive electrode plate, a separator, and a negative electrode plate, where the positive electrode plate includes a positive electrode active material layer, and the negative electrode plate includes a negative electrode active material layer. Observed along a thickness direction of the electrochemical device, in a width direction of the negative electrode plate, the negative electrode active material layer includes two protruding regions not overlapping with the positive electrode active material layer on two sides. The electrochemical device further includes an electrolyte, the electrolyte includes a polynitrile compound, and the polynitrile compound includes a compound represented by formula I-A:

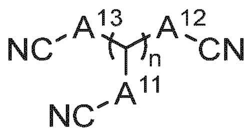 formula I-A;

where

in formula I-A, $A^{11}$, $A^{12}$, and $A^{13}$ are independently selected from one of formula (I-A1) or formula (I-A2):

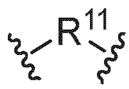 formula (I-A1) or 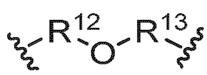 formula (I-A2);

and

in formula I-A, n is a positive integer from 1 to 8, and for example, n may be 1, 2, 3, 4, 5, 6, 7, or 8, where when multiple $A^{11}$ are present, the multiple $A^{11}$ may be the same or different; $R^{11}$, $R^{11}$, and $R^{13}$ are independently selected from a covalent single bond, substituted or unsubstituted $C_1$-$C_{10}$ alkylene group, substituted or unsubstituted $C_2$-$C_{10}$ alkenylene group, substituted or unsubstituted $C_2$-$C_{10}$ alkynylene group, substituted or unsubstituted $C_6$-$C_{10}$ arylene group, substituted or unsubstituted $C_3$-$C_{10}$ cycloalkylene group, or substituted or unsubstituted $C_1$-$C_{10}$ heterocyclene group, where when the foregoing groups are substituted, a substituent group is selected from halogen; and the heterocyclene group includes heteroalicyclene group and heterocycloarylene group, and heteroatom in the heterocyclene group is selected from at least one of O, N, S, or Si; where 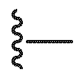 represents a bonding site with an adjacent atom. In this application, the halogen is selected from F or Cl.

The applicant has found through extensive experiments that when based on a mass of the electrolyte, a mass percentage of the polynitrile compound is 0.01% to 8%, and width of the protruding region is 0.5 mm to 2 mm, comprehensive performance of the electrochemical device is significantly improved. A possible reason is that when the polynitrile compound is added to the electrolyte, an appropriate amount of the polynitrile compound can efficiently exert effect on a positive electrode, stabilize the positive electrode plate in a charged state, and reduce dissolution of transition metal ions in a high potential environment, thereby suppressing increase in negative electrode impedance. However, a protruding region with an inappropriate length (for example, greater than 2 mm) leads to accumulation of part of the polynitrile compound in the protruding region, which easily causes abnormal lithium precipitation at the boundary of the protruding region, increasing the negative electrode impedance. Meanwhile, the polynitrile compound cannot fully exert effect on the positive electrode active material layer, which results in deposition of part of transition metal ions dissolved from the positive electrode on a surface of a negative electrode, further increasing the negative electrode impedance. In addition, due to the abnormal lithium precipitation in the protruding region, the degree of lithium deintercalation from the positive electrode increases during cycling of the electrochemical device, which exacerbates damage to the positive electrode material and increases gas generation during floating charge of the electrochemical device, leading to increased floating charge thickness swelling rate and deteriorated floating charge performance of the electrochemical device. In this application, "positive electrode" can be understood as "positive electrode plate", and "negative electrode" can be understood as "negative electrode plate".

Based on a mass of the electrolyte, a mass percentage N% of the polynitrile compound is 0.01% to 8%. For example, N% may be 0.01%, 1%, 2%, 3%, 4%, 5%, 6%, 7%, 8%, or any value within a range defined by any two of these values. When the mass percentage N% of the polynitrile compound is less than 0.01%, the polynitrile compound cannot exert effect. However, when N% is greater than 8%, in addition to forming a stable cathode electrolyte interface (CEI) film on the surface of the positive electrode plate by the polynitrile compound, the excess polynitrile compound continues to form the CEI film on the surface of the positive electrode plate, which consumes active lithium and forms an excessively thick CEI film, affecting the floating charge performance of the electrochemical device. Based on the mass of the electrolyte, the mass percentage N% of the polynitrile compound is preferably 0.5% to 5%.

Width of the protruding region is M mm, where $0.5 \leq M \leq 2$. For example, the width of the protruding region is 0.5 mm, 1 mm, 1.5 mm, 2 mm, or any value within a range defined by any two of these values. When the width of the protruding region is less than 0.5 mm, the width of the protruding region is excessively small, causing the electrolyte to accumulate in the protruding region to form a solid electrolyte film with high impedance in that region. Therefore, during charging of the electrochemical device, as lithium ions diffuse from a body region (an overlapping region of the positive electrode plate and the negative electrode plate) to the protruding region, the risk of lithium precipitation in the protruding region increases, and side reactions caused by lithium precipitation in that region result in a greater negative electrode impedance increase rate, leading to increased floating charge thickness swelling rate and deteriorated floating charge performance of the electrochemical device.

The width M mm of the protruding region and the mass percentage N% of the polynitrile compound satisfy $0.01 \leq M \times N < 10$. For example, the value of $M \times N$ may be 0.01, 0.5, 1, 2, 2.6, 3, 3.9, 4, 5, 6, 7, 8, 9, 9.99, or any value within a range defined by any two of these values.

Controlling the width M mm of the protruding region, the mass percentage N% of the polynitrile compound, and the value of $M \times N$ within the ranges of this application allows the protruding region and the polynitrile compound in the electrolyte to form a beneficial interaction, which reduces free polynitrile compound in the electrolyte and side reactions in the protruding region, thereby further reducing the risk of lithium precipitation in the protruding region of the negative electrode plate. When applied to a system with a high charge cut-off voltage (for example, 4.5 V to 4.8 V), this method can suppress increase in the negative electrode impedance and improve the floating charge performance of the electrochemical device.

Preferably, the width M mm of the protruding region and the mass percentage N% of the polynitrile compound satisfy $0.5 < M \times N \leq 7$. More preferably, $2.6 \leq M \times N \leq 3.9$. Controlling the value of $M \times N$ within the foregoing preferred range allows for lower negative electrode impedance increase rate and better floating charge performance of the electrochemical device.

In an embodiment of this application, at least two of $A^{11}$, $A^{12}$, and $A^{13}$ are selected from formula (I-A2).

Preferably, the compound represented by formula I-A includes at least one of compounds represented by formulas (I-1) to (I-20):

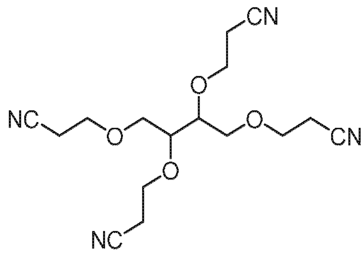

formula (I-1),

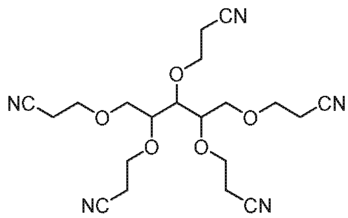

formula (I-2),

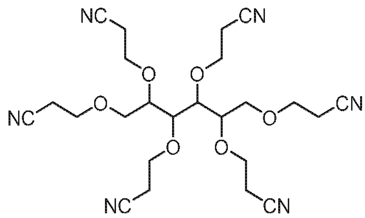

formula (I-3),

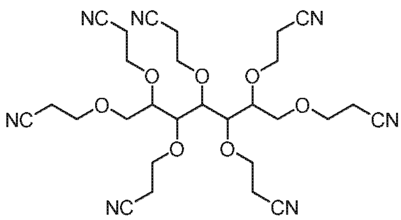

formula (I-4),

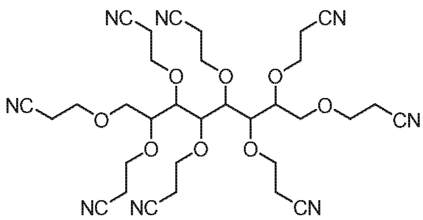

formula (I-5),

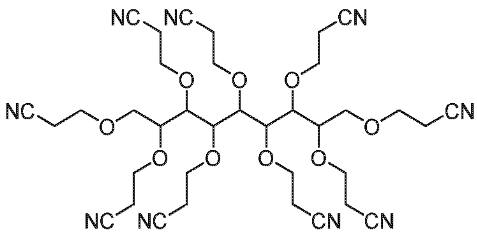

formula (I-6),

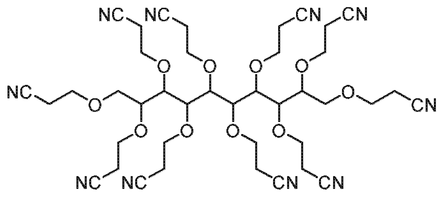
formula (I-7),

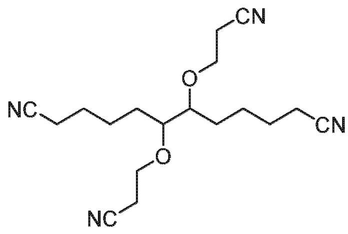
formula (I-8),

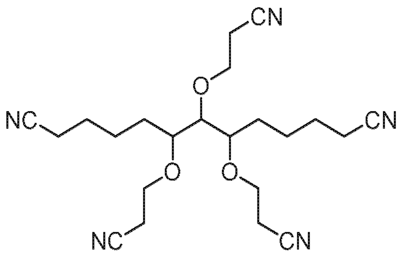
formula (I-9),

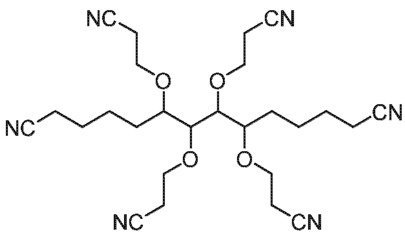
formula (I-10),

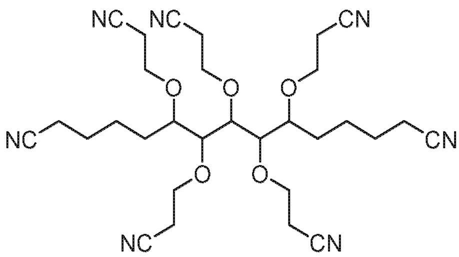
formula (I-11),

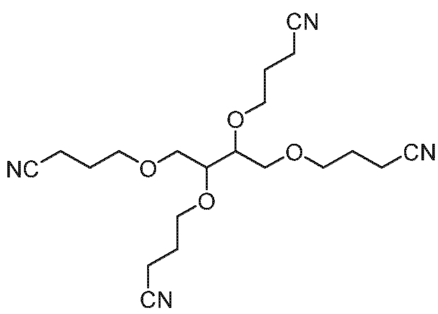
formula (I-12),

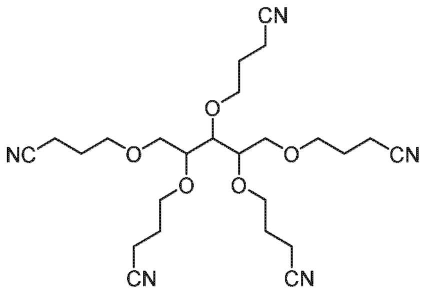

formula (I-13),

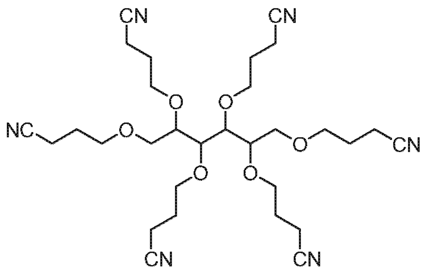

formula (I-14),

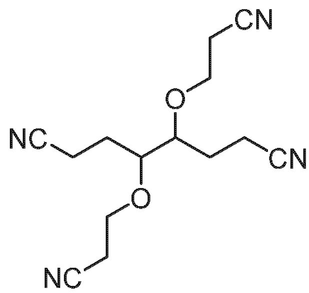

formula (I-15),

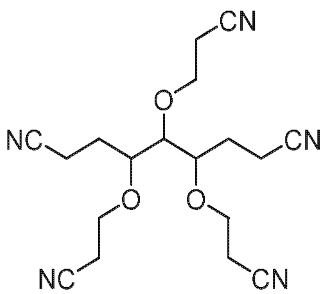

formula (I-16),

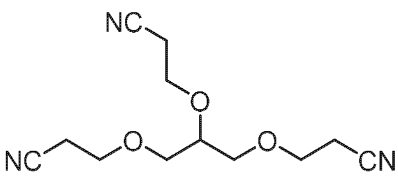

formula (I-17),

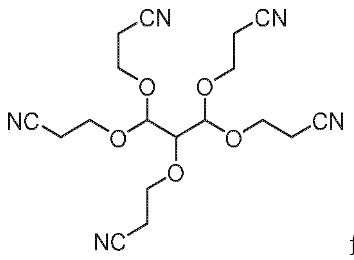

formula (I-18),

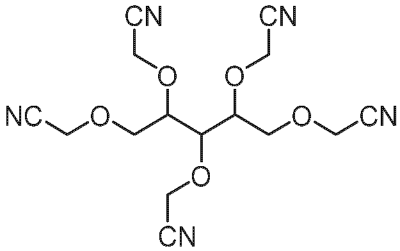

formula (I-19), or

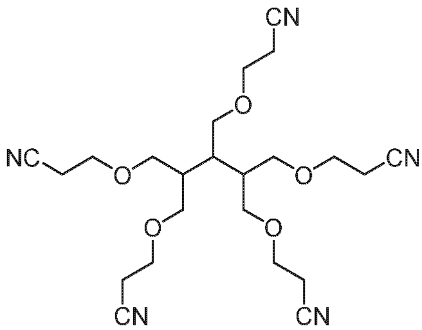

formula (I-20).

Preferably, the compound represented by formula I-A includes at least one of 1,2,3-tris(2-cyanoethoxy)propane or 1,3,6-hexanetricarbonitrile.

In an embodiment of this application, the electrolyte further includes a phosphonitrile compound, and the phosphonitrile compound includes a compound represented by formula II-A:

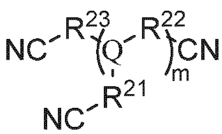

formula II-A;

where

in formula II-A, Q is independently selected from formula (II-A1) or formula (II-A2):

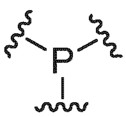

formula (II-A1) or

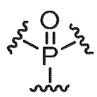

formula (II-A2);

in formula II-A, m is selected from 1 or 2, each Q may be the same or different, and each $R^{22}$ may be the same or different; $R^{21}$, $R^{22}$, and $R^{23}$ are independently selected from a covalent single bond, substituted or unsubstituted $C_1$-$C_{10}$ alkylene group, substituted or unsubstituted $C_2$-$C_{10}$ alkenylene group, substituted or unsubstituted $C_2$-$C_{10}$ alkynylene group, substituted or unsubstituted $C_6$-$C_{10}$ arylene group, substituted or unsubstituted $C_3$-$C_{10}$ cycloalkylene group, or substituted or unsubstituted $C_1$-$C_{10}$ heterocyclene group, where when the foregoing groups are substituted, a substituent group is selected from halogen; and the heterocyclene group includes heteroalicyclene group and heterocycloarylene group, and heteroatom in the heterocyclene group is selected from at least one of O, N,

S, or Si; where 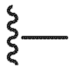 represents a bonding site with an adjacent atom. Based on the mass of the electrolyte, a mass percentage P% of the phosphonitrile compound is 0.01% to 6%, and preferably 1% to 2%. For example, P% may be 0.01%, 1%, 2%, 3%, 4%, 5%, 6%, or any value within a range defined by any two of these values.

When the phosphonitrile compound of this application is further added to the electrolyte and the mass percentage P% of the phosphonitrile compound is controlled within the range of this application, the phosphonitrile compound can decompose at high temperature to release P radicals, which can combine with O radicals released from the positive electrode, reducing oxidative decomposition of the electrolyte at high temperature, thereby improving thermal stability of the electrochemical device and improving the floating charge performance and hot-box performance. Controlling the mass percentage P% of the phosphonitrile compound within the range of this application allows for excellent floating charge performance and hot-box performance of the electrochemical device.

Preferably, the compound represented by formula II-A includes at least one of compounds represented by formulas (II-1) to (II-21):

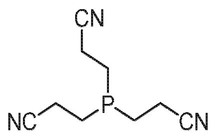 formula (II-1),

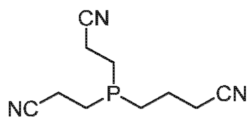 formula (II-2),

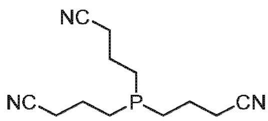 formula (II-3),

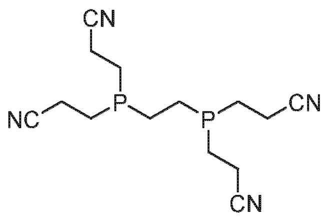 formula (II-4),

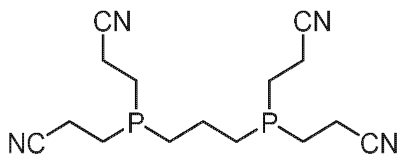 formula (II-5),

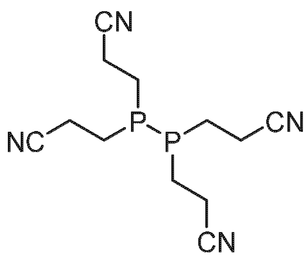 formula (II-6),

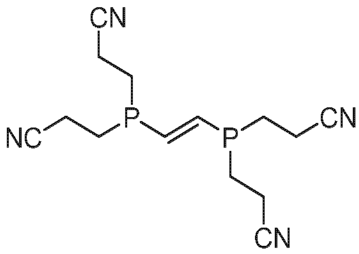

formula (II-7),

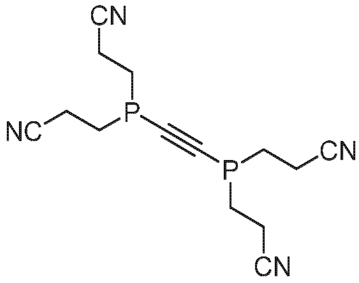

formula (II-8),

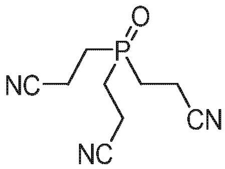

formula (II-9),

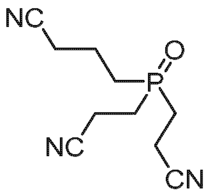

formula (II-10),

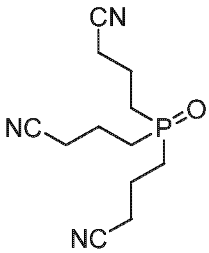

formula (II-11),

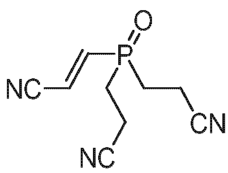

formula (II-12),

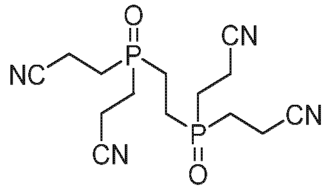
formula (II-13),

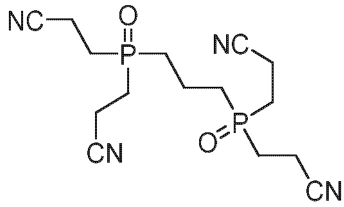
formula (II-14),

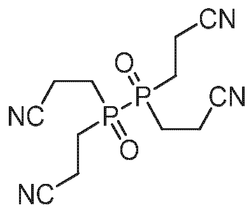
formula (II-15),

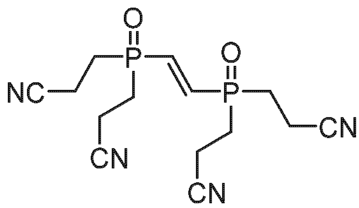
formula (II-16),

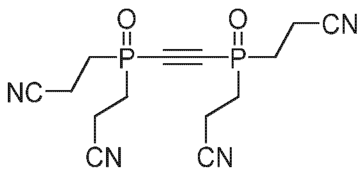
formula (II-17),

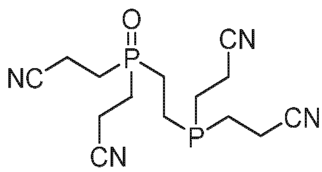
formula (II-18),

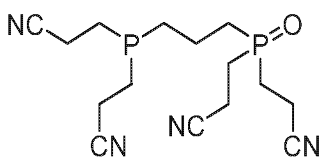
formula (II-19),

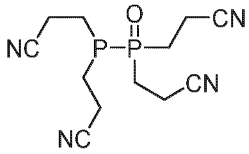 formula (II-20), or

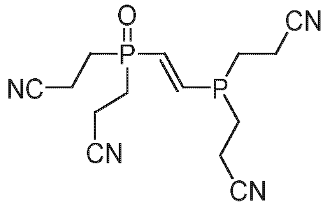 formula (II-21).

In an embodiment of this application, the mass percentage N% of the polynitrile compound and the mass percentage P% of the phosphonitrile compound satisfy $0.1 \leq N+P \leq 10$. For example, the value of N+P (%) may be 0.1, 1, 2, 3, 4, 5, 6, 7, 8, 9, 10, or any value within a range defined by any two of these values. Controlling the value of N+P within the foregoing range is more conducive to improving the floating charge performance and safety performance of the electrochemical device. Preferably, $2 \leq N+P \leq 8$. More preferably, $2.5 \leq N+P \leq 6.5$.

In an embodiment of this application, the electrolyte further includes linear carboxylate, and based on the mass of the electrolyte, a mass percentage X% of the linear carboxylate is 1% to 60%. For example, X% may be 1%, 5%, 10%, 20%, 30%, 40%, 50%, 60%, or any value within a range defined by any two of these values. Adding the linear carboxylate to the electrolyte can increase a transmission rate of lithium ions in the electrolyte, and the linear carboxylate can protect the positive electrode plate during reaction between the positive electrode plate and the electrolyte, thereby improving the floating charge performance of the electrochemical device. However, when the mass percentage X% of the linear carboxylate is greater than 60%, side reaction products generated from reactions of the linear carboxylate on the surface of the positive electrode plate increase, which in turn leads to a greater increase in the negative electrode impedance, affecting the floating charge performance of the electrochemical device. Controlling the mass percentage X% of the linear carboxylate within the range of this application is more conducive to improving the floating charge performance of the electrochemical device.

In an embodiment of this application, the linear carboxylate includes at least one of ethyl acetate, propyl acetate, butyl acetate, ethyl propionate, propyl propionate, butyl butyrate, methyl butyrate, methyl isovalerate, propyl hexanoate, or isobutyl acetate. Using the foregoing types of linear carboxylate is more conducive to improving the floating charge performance of the electrochemical device.

In an embodiment of this application, the mass percentage N% of the polynitrile compound and the mass percentage X% of the linear carboxylate satisfy $N/X \geq 0.01$. For example, the value of N/X may be 0.01, 0.033, 0.05, 0.1, 0.2, 0.4, or any value within a range defined by any two of these values. When the value of N/X is controlled within the range of this application, the polynitrile compound preferentially forms a CEI film on the surface of the positive electrode plate, improving the floating charge performance of the electrochemical device, and the linear carboxylate added to the electrolyte can reduce viscosity of the electrolyte and suppress increase in the negative electrode impedance. The synergistic effect of the polynitrile compound and the linear carboxylate effectively improves the floating charge performance of the electrochemical device. Preferably, $0.05 \leq N/X \leq 0.4$. More preferably, $0.1 \leq N/X \leq 0.25$.

In an embodiment of this application, the electrolyte further includes a phosphonitrile compound and linear carboxylate; where based on the mass of the electrolyte, the mass percentage N% of the polynitrile compound, a mass percentage P% of the phosphonitrile compound, and a mass percentage X% of the linear carboxylate satisfy $X/(5N+P) \leq 20$. Preferably, $0.4 \leq X/(5N+P) \leq 10$. For example, the value of X/(5N+P) may be 0.1, 0.4, 0.45, 0.91, 1.82, 3.64, 5.45, 10, 20, or any value within a range defined by any two of these values. Adding all of the polynitrile compound, the phosphonitrile compound, and the linear carboxylate to the electrolyte and controlling the value of X/(5N+P) within the range of this application not only effectively improve the floating charge performance of the electrochemical device but also effectively improve the safety performance of the electrochemical device. More preferably, $0.91 \leq X/(5N+P) \leq 3.64$.

In an embodiment of this application, the electrolyte further includes a lithium salt. The lithium salt is not particularly limited in this application, provided that the objectives of this application can be achieved. For example, the lithium salt may include at least one of $LiPF_6$, $LiBF_4$, $LiAsF_6$, $LiClO_4$, $LiB(C_6H_5)_4$, $LiCF_3SO_3$, $LiN(SO_2CF_3)_2$, $LiC(SO_2CF_3)_3$, $LiSiF_6$, LiBOB (lithiumbis(oxalato)borate), or LiDFOB (lithium difluoro(oxalato)borate), and preferably $LiPF_6$. Mass percentage of the lithium salt in the electrolyte is not particularly limited in this application, provided that the objectives of this application can be achieved. For example, based on the mass of the electrolyte, the mass percentage of the lithium salt is 8% to 15%.

The electrolyte of this application further includes a non-aqueous solvent. The non-aqueous solvent is not particularly limited in this application, provided that the objectives of this application can be achieved. For example, the non-aqueous solvent may include at least one of a carbonate compound, a carboxylate compound, an ether compound, or another organic solvent. The carbonate compound may include but is not limited to at least one of a linear carbonate compound, a cyclic carbonate compound, or a fluorocarbonate compound. The linear carbonate compound may include but is not limited to at least one of dimethyl carbonate (DMC), diethyl carbonate (DEC), dipropyl carbonate (DPC), methyl propyl carbonate (MPC), ethyl propyl carbonate (EPC), or ethyl methyl carbonate (EMC). The cyclic carbonate compound may include but is not limited to at least one of ethylene carbonate (EC), propylene carbonate (PC), butylene carbonate (BC), or vinyl ethylene carbonate (VEC). The fluorocarbonate compound may include but is not limited to at least one of fluoroethylene carbonate (FEC), 1,2-difluoroethylene carbonate, 1,1-difluoroethylene carbonate, 1,1,2-trifluoroethylene carbonate, 1,1,2,2-tetrafluoroethylene carbonate, 1-fluoro-2-methylethylene carbonate, 1-fluoro-1-methylethylene carbonate, 1,2-difluoro-1-methylethylene carbonate, 1,1,2-trifluoro-2-methylethylene carbonate, or trifluoromethylethylene carbonate. The carboxylate compound may include but is not limited to at least one of n-propyl acetate, tert-butyl acetate, or methyl propionate. The ether compound may include but is not limited to at least one of dibutyl ether, tetraglyme, diglyme, 1,2-dimethoxyethane, 1,2-diethoxyethane, ethoxymethoxyethane, 2-methyltetrahydrofuran, or tetrahydrofuran. The another organic solvent may include but is not limited to at least one of dimethyl sulfoxide, 1,2-dioxolane, sulfolane, methyl sulfolane, 1,3-dimethyl-2-imidazolidinone, N-methyl-2-pyrrolidone, formamide, dimethylformamide, acetonitrile, trimethyl phosphate, triethyl phosphate, trioctyl phosphate, or phosphate ester. Based on the mass of the electrolyte, a mass percentage of the non-aqueous solvent is 5% to 70%, for example, 5%, 10%, 15%, 20%, 25%, 30%, 35%, 40%, 45%, 50%, 55%, 60%, 65%, 70%, or any value within a range defined by any two of these values.

The positive electrode plate is not particularly limited in this application, provided that the objectives of this application can be achieved. For example, the positive electrode plate includes a positive electrode current collector and a positive electrode active material layer. The positive electrode current collector is not particularly limited in this application, provided that the objectives of this application can be achieved. For example, the positive electrode current collector may include aluminum foil, aluminum alloy foil, or the like. The positive electrode active material layer in this application includes a positive electrode material. Type of the positive electrode material is not particularly limited in this application, provided that the objectives of this application can be achieved. For example, the positive electrode material may include at least one of lithium nickel cobalt manganate (811, 622, 523, 111), lithium nickel cobalt aluminate, lithium iron phosphate, lithium-rich manganese-based material, lithium cobalt oxide ($LiCoO_2$), lithium manganese oxide, lithium manganese iron phosphate, or lithium titanate. In this application, the positive electrode material may further include a non-metal element. For example, the non-metal element includes at least one of fluorine, phosphorus, boron, chlorine, silicon, or sulfur. These elements can further improve stability of the positive electrode material. In this application, thicknesses of the positive electrode current collector and the positive electrode active material layer are not particularly limited, provided that the objectives of this application can be achieved. For example, the thickness of the positive electrode current collector is 5 μm to 20 μm, and preferably 6 μm to 18 μm. The thickness of the one-sided positive electrode active material layer is 30 μm to 120 μm. In this application, the positive electrode active material layer may be disposed on one surface of the positive electrode current collector in its thickness direction, or on two surfaces of the positive electrode current collector in its thickness direction. It should be noted that the "surface" herein may be an entire region or a partial region of the positive electrode current collector. This is not particularly limited in this application, provided that the objectives of this application can be achieved. Optionally, the positive electrode plate may further include a conductive layer. The conductive layer is located between the positive electrode current collector and the positive electrode active material layer. Composition of the conductive layer is not particularly limited, and the conductive layer may be a conductive layer commonly used in the art. The conductive layer includes a conductive agent and a binder.

In an embodiment of this application, an insulating layer is provided on a surface of the positive electrode current collector, the insulating layer includes a binder and an inorganic oxide containing element aluminum or an inorganic nitride containing element aluminum, and in a width direction of the positive electrode plate, the insulating layer is disposed on two sides of the positive electrode active material layer. In an embodiment of this application, width of the insulating layer is 1.5 mm to 5 mm. The insulating layer being disposed on two sides of the positive electrode active material layer can improve stability of a positive electrode active material near the protruding region to some extent, reducing the impedance in the region at the boundary of the interface of the negative electrode and reducing the risk of lithium precipitation in the protruding region, thereby further improving the floating charge performance and safety performance of the electrochemical device.

The negative electrode plate is not particularly limited in this application, provided that the objectives of this application can be achieved. For example, the negative electrode plate includes a negative electrode current collector and a negative electrode active material layer. The negative electrode current collector is not particularly limited in this application, provided that the objectives of this application can be achieved. For example, the negative electrode current collector may include copper foil, copper alloy foil, nickel foil, titanium foil, nickel foam, copper foam, or the like. The negative electrode active material layer in this application includes a negative electrode material. Type of the negative

electrode material is not particularly limited in this application, provided that the objectives of this application can be achieved. For example, the negative electrode material may include at least one of natural graphite, artificial graphite, mesocarbon microbeads (MCMB), hard carbon, soft carbon, silicon, a silicon-carbon composite, $SiO_x$ ($0 < x < 2$), or lithium metal. In this application, thicknesses of the negative electrode current collector and the negative electrode active material layer are not particularly limited, provided that the objectives of this application can be achieved. For example, the thickness of the negative electrode current collector is 6 μm to 10 μm, and the thickness of the one-sided negative electrode active material layer is 30 μm to 130 μm. In this application, the negative electrode active material layer may be disposed on one surface of the negative electrode current collector in its thickness direction, or on two surfaces of the negative electrode current collector in its thickness direction. It should be noted that the "surface" herein may be an entire region or a partial region of the negative electrode current collector. This is not particularly limited in this application, provided that the objectives of this application can be achieved. Optionally, the negative electrode plate may further include a conductive layer. The conductive layer is located between the negative electrode current collector and the negative electrode active material layer. Composition of the conductive layer is not particularly limited, and the conductive layer may be a conductive layer commonly used in the art. The conductive layer includes a conductive agent and a binder.

The conductive agent and the binder are not particularly limited, provided that the objectives of this application can be achieved. For example, the conductive agent may include at least one of conductive carbon black (Super P), carbon nanotubes (CNTs), carbon nanofiber, flake graphite, acetylene black, carbon black, Ketjen black, carbon dots, or graphene. For example, the binder may include at least one of polypropylene alcohol, sodium polyacrylate, potassium polyacrylate, lithium polyacrylate, polyimide, polyimide, polyamide-imide, styrene-butadiene rubber (SBR), polyvinyl alcohol (PVA), polyvinylidene fluoride, polyvinylidene difluoride (PVDF), polytetrafluoroethylene (PTFE), polyvinyl butyral (PVB), water-borne acrylic resin, carboxymethyl cellulose (CMC), or sodium carboxymethyl cellulose (CMC-Na).

The separator is not particularly limited in this application, provided that the objectives of this application can be achieved. For example, the separator may include a substrate layer and a surface treatment layer. The substrate layer may be a non-woven fabric, film, or composite film having a porous structure, and material of the substrate layer may include at least one of polyethylene, polypropylene, polyethylene terephthalate, or polyimide. Optionally, the substrate layer may be a polypropylene porous film, a polyethylene porous film, a polypropylene non-woven fabric, a polyethylene non-woven fabric, or a polypropylene-polyethylene-polypropylene porous composite film. Optionally, the surface treatment layer is provided on at least one surface of the substrate layer, and the surface treatment layer may be a polymer layer, an inorganic substance layer, or a layer formed by mixing a polymer and an inorganic substance. For example, the inorganic substance layer includes inorganic particles and a binder. The inorganic particle is not particularly limited, and for example, may be selected from at least one of aluminum oxide, silicon oxide, magnesium oxide, titanium oxide, hafnium oxide, tin oxide, ceria oxide, nickel oxide, zinc oxide, calcium oxide, zirconium oxide, yttrium oxide, silicon carbide, boehmite, aluminum hydroxide, magnesium hydroxide, calcium hydroxide, or barium sulfate. The binder is not particularly limited, and for example, may be selected from at least one of polyvinylidene fluoride, vinylidene fluoride-hexafluoropropylene copolymer, polyamide, polyacrylonitrile, polyacrylate ester, polyacrylic acid, polyacrylate salt, polyvinylpyrrolidone, polyvinyl ether, polymethyl methacrylate, polytetrafluoroethylene, or polyhexafluoropropylene. The polymer layer includes a polymer, and material of the polymer includes at least one of polyamide, polyacrylonitrile, acrylate polymer, polyacrylic acid, polyacrylate salt, polyvinylpyrrolidone, polyvinyl ether, polyvinylidene fluoride, or poly(vinylidene fluoride-hexafluoropropylene).

The electrochemical device of this application is not particularly limited, and may include any device in which electrochemical reactions take place. In some embodiments, the electrochemical device may include but is not limited to a lithium metal secondary battery, a lithium-ion battery (lithium-ion secondary battery), a lithium polymer secondary battery, or a lithium-ion polymer secondary battery.

A preparation process of the electrochemical device is well known to persons skilled in the art, and is not particularly limited in this application. For example, the preparation process may include but is not limited to the following steps: a positive electrode plate, a separator, and a negative electrode plate are stacked in sequence and go through operations such as winding and folding as needed to obtain an electrode assembly with a winding structure, the electrode assembly is put into an outer package, an electrolyte is injected into the outer package, and the outer package is sealed to obtain an electrochemical device; or a positive electrode plate, a separator, and a negative electrode plate are stacked in sequence, four corners of the entire laminated structure are fixed with tapes to obtain an electrode assembly with a laminated structure, the electrode assembly is put into an outer package, an electrolyte is injected into the outer package, and the outer package is sealed to obtain an electrochemical device. In addition, an overcurrent prevention element, a guide plate, and the like may also be put into the outer package as needed, so as to prevent pressure increase, overcharge, and overdischarge inside the electrochemical device.

A second aspect of this application provides an electronic device including the electrochemical device according to any one of the foregoing embodiments. Therefore, the electronic device also has good floating charge performance.

The electronic device of this application is not particularly limited, and may include but is not limited to the following types: a notebook computer, a pen-input computer, a mobile computer, an electronic book player, a portable telephone, a portable fax machine, a portable copier, a portable printer, a stereo headset, a video recorder, a liquid crystal

television, a portable cleaner, a portable CD player, a mini-disc, a transceiver, an electronic notebook, a calculator, a storage card, a portable recorder, a radio, a standby power source, a motor, an automobile, a motorcycle, a motor bicycle, a bicycle, a lighting appliance, a toy, a game console, a clock, an electric tool, a flash lamp, a camera, a large household battery, or a lithium-ion capacitor.

This application provides an electrochemical device and an electronic device including the same. The electrochemical device includes a positive electrode plate, a separator, and a negative electrode plate, where the positive electrode plate includes a positive electrode active material layer, and the negative electrode plate includes a negative electrode active material layer. Observed along a thickness direction of the electrochemical device, in a width direction of the negative electrode plate, the negative electrode active material layer includes two protruding regions not overlapping with the positive electrode active material layer on two sides, and width M mm of the protruding region is 0.5 mm to 2 mm. The electrochemical device further includes an electrolyte, the electrolyte includes a polynitrile compound, and the polynitrile compound includes a compound represented by formula I-A. Based on a mass of the electrolyte, a mass percentage N% of the polynitrile compound is 0.01% to 8%. The width M mm of the protruding region and the mass percentage N% of the polynitrile compound satisfy $0.01 \leq M \times N < 10$. With the width M mm of the protruding region, the mass percentage N% of the polynitrile compound, and the relation $M \times N$ of the width M of the protruding region and the mass percentage N% of the polynitrile compound satisfying the foregoing ranges, the floating charge performance of the electrochemical device is improved.

## BRIEF DESCRIPTION OF DRAWINGS

To describe the technical solutions in embodiments of this application more clearly, the following briefly describes the accompanying drawings required for describing some embodiments. Apparently, the accompanying drawings in the following descriptions show merely some embodiments of this application, and persons of ordinary skill in the art may still derive other drawings from these accompanying drawings.

FIG. 1 is a schematic diagram of a position relationship between a positive electrode plate and a negative electrode plate according to an embodiment of this application.

Reference signs in the specific embodiments are described as follows:

10. negative electrode plate, 20. positive electrode plate, and 30. protruding region.

## DESCRIPTION OF EMBODIMENTS

To make the objectives, technical solutions, and advantages of this application more comprehensible, the following further describes this application in detail with reference to accompanying drawings and embodiments. Apparently, the described embodiments are merely some rather than all of the embodiments of this application. All other embodiments obtained by persons of ordinary skill in the art based on some embodiments of this application shall fall within the protection scope of this application.

It should be noted that in specific embodiments of this application, an example in which a lithium-ion battery is used as an electrochemical device is used to illustrate this application. However, the electrochemical device of this application is not limited to the lithium-ion battery.

FIG. 1 is a schematic diagram of a position relationship between a positive electrode plate and a negative electrode plate according to an embodiment of this application. As shown in FIG. 1, in a width direction of a negative electrode plate 10, the negative electrode plate 10 includes two opposite protruding regions 30 extending beyond a positive electrode plate 20, and width M mm of the protruding region 30 is 0.5 mm to 2 mm. It should be noted that to show the position relationship between the positive electrode plate and the negative electrode plate, a separator between the positive electrode plate and the negative electrode plate is not shown in FIG. 1.

The following describes this application more specifically by using examples and comparative examples. Various tests and evaluations are performed according to the following methods.

### Test methods and equipment

### Negative electrode impedance increase rate test

A three-electrode lithium-ion battery was charged to 50% SOC (state of charge) and then subjected to a lithium plating test: the positive electrode of the test line was connected to the positive electrode tab of the three-electrode lithium-ion battery, the negative electrode of the test line was connected to the copper wire of the three-electrode lithium-ion battery, and the battery was charged at a current of 0.02 mA for 2 h to complete the lithium plating. At 25°C, an electrochemical workstation was used, and the negative electrode tab of the lithium-ion battery and the three-electrode copper wire were connected, with the frequency adjusted to 5 mHz to 500,000 Hz and the perturbation voltage set to 5 mV,

to test the alternating current impedance of the negative electrode.

Impedance test before and after cycling: the negative electrode impedance before cycling was tested using the foregoing method; and the lithium-ion battery was subjected to 500 cycles under the following conditions, and the negative electrode impedance after 500 cycles was tested.

At 25°C, the lithium-ion battery was charged to 4.25 V at a constant current of 1.2C, charged to a current of 0.6C at a constant voltage of 4.25 V, then charged to 4.5 V at a constant current of 0.6C, charged to 0.05C at a constant voltage, and discharged to 3.0 V at a constant current of 0.5C. This was one cycle.

Negative electrode impedance increase rate = (negative electrode impedance after cycling - negative electrode impedance before cycling)/negative electrode impedance before cycling×100%.

<Preparation of three-electrode lithium-ion battery>

A positive electrode plate, a separator, and a negative electrode plate were sequentially stacked so that the separator was located between the positive electrode plate and the negative electrode plate for separation. A 20 μm thick and 120 mm long copper wire acid-washed with concentrated sulfuric acid for 20 min was used, 80 mm of the copper wire was wrapped with the separator and then placed between the positive electrode plate and the separator, and the remaining 40 mm of the copper wire extended out from the end surface of the positive electrode plate. Then, the positive electrode plate, the separator, and the negative electrode plate were wound to obtain an electrode assembly. After the positive electrode tab and the negative electrode tab were welded, the electrode assembly was placed in an outer package aluminum-plastic film, and the electrolyte was injected into the outer package aluminum-plastic film to infiltrate the electrode assembly, followed by processes such as vacuum sealing, standing, formation, shaping, and capacity testing, to obtain a three-electrode lithium-ion battery.

**Floating charge thickness swelling rate test**

At 25°C, an initial thickness D1 of the lithium-ion battery was measured. At 45°C, the lithium-ion battery was left standing for 1 h, discharged to 3.0 V at a constant current of 0.2C, left standing for 5 min, charged to 4.25 V at a constant current of 1.1C, then charged to 0.7C at a constant voltage, charged to 4.5 V at a constant current of 0.7C, and charged at a constant voltage of 4.5 V for 1500 h. After 1500 h, the test was stopped, a thickness D2 of the lithium-ion battery was recorded, and the thickness swelling rate was calculated according to the following formula and used as an indicator for evaluating the floating charge performance of the lithium-ion battery.

$$\text{Thickness swelling rate} = (D2 - D1)/D1 \times 100\%$$

**Hot-box test**

At 25°C, lithium-ion batteries were charged to 5.0 V at a constant current of 0.7C, and then charged at a constant voltage of 5.0 V for 8 h, and changes in the lithium-ion batteries were monitored. Lithium-ion batteries that did not catch fire or explode passed the test. Ten samples were tested for each example and comparative example, and the number of lithium-ion batteries that passed the test was recorded and used as an indicator for evaluating the safety performance of the lithium-ion battery.

**Example 1-1**

<Preparation of electrolyte>

In an argon atmosphere glove box with a water content of < 10 ppm, EC, PC, and DMC were mixed at a mass ratio of 30:25:45 to obtain a base solvent, then lithium salt $LiPF_6$ and a polynitrile compound formula (I-2) were added to the base solvent, and the mixture was stirred to uniformity to obtain an electrolyte. Based on a mass of the electrolyte, a mass percentage of the $LiPF_6$ was 12%, a mass percentage N% of the formula (I-1) was 0.01%, and the remaining was the base solvent.

<Preparation of positive electrode plate>

A positive electrode material $LiCoO_2$, a conductive agent conductive carbon black, and a binder PVDF were mixed at a mass ratio of 95:2:3, N-methylpyrrolidone (NMP) was added, and the mixture was stirred to uniformity using a

vacuum stirrer to produce a positive electrode slurry, where a solid content of the positive electrode slurry was 70wt%. The positive electrode slurry was uniformly applied onto one surface of a 12 μm thick positive electrode current collector aluminum foil, and the aluminum foil was dried at 85°C for 4 h to obtain a positive electrode plate coated with a 130 μm thick and 74 mm wide positive electrode active material layer on one surface. The same steps were repeated on another surface of the aluminum foil to obtain a positive electrode plate coated with positive electrode active material layers on two surfaces. Then, the positive electrode plate was dried in vacuum at 85°C for 4 h, followed by cold pressing, cutting, and slitting, to obtain a 74 mm×867 mm positive electrode plate.

<Preparation of negative electrode plate>

A negative electrode material graphite, a binder styrene-butadiene rubber, and a negative electrode thickener sodium carboxymethyl cellulose were mixed at a mass ratio of 95:2:3, deionized water was added, and the mixture was stirred to uniformity using a vacuum stirrer to produce a negative electrode slurry, where a solid content of the negative electrode slurry was 75wt%. The negative electrode slurry was uniformly applied onto one surface of a 12 μm thick negative electrode current collector copper foil, and the copper foil was dried at 85°C for 4 h to obtain a negative electrode plate coated with a 130 μm thick and 76.6 mm wide negative electrode active material layer on one surface. The same steps were repeated on another surface of the aluminum foil to obtain a negative electrode plate coated with negative electrode active material layers on two surfaces. Then, the negative electrode plate was dried in vacuum at 85°C for 4 h, followed by cold pressing, cutting, and slitting, to obtain a 76.6 mm×875 mm negative electrode plate.

<Preparation of separator>

A 14 μm thick polyethylene (PE) film (provided by Celgard) was used.

<Preparation of lithium-ion battery>

The prepared positive electrode plate, separator, and negative electrode plate were sequentially stacked so that the separator was located between the positive electrode plate and the negative electrode plate for separation. Then, the resulting stack was wound to obtain an electrode assembly. The electrode assembly was placed in an outer package aluminum-plastic film and dried in a vacuum oven at 85°C for 12 h to remove moisture, and the prepared electrolyte was injected, followed by processes such as vacuum sealing, standing, formation, and shaping, to obtain a lithium-ion battery. Width M mm of a protruding region was 1.3 mm.

**Examples 1-2 to 1-14**

These examples were the same as Example 1-1 except that the related preparation parameters were adjusted according to Table 1.

**Example 1-15**

This example was the same as Example 1-1 except that the related preparation parameters were adjusted according to Table 1 and the positive electrode plate was prepared according to the following method.

<Preparation of positive electrode plate>

A positive electrode material $LiCoO_2$, a conductive agent conductive carbon black, and a binder PVDF were mixed at a mass ratio of 95:2:3, NMP was added, and the mixture was stirred to uniformity using a vacuum stirrer to produce a positive electrode slurry, where a solid content of the positive electrode slurry was 70wt%. Aluminum oxide and the binder PVDF at a mass ratio of 90:10 were mixed with the NMP to produce an insulating layer slurry. The positive electrode slurry and the insulating layer slurry were uniformly applied onto one surface of a 12 μm thick positive electrode current collector aluminum foil, and the aluminum foil was dried at 85°C for 4 h to obtain a positive electrode plate sequentially coated with a 130 μm thick and 74 mm wide positive electrode active material layer and a 130 μm thick and 2 mm wide insulating layer on one surface. The same steps were repeated on another surface of the aluminum foil to obtain a positive electrode plate sequentially coated with positive electrode active material layers and insulating layers on both surfaces. Then, the positive electrode plate was dried in vacuum at 85°C for 4 h, followed by cold pressing, cutting, and slitting, to obtain a 78 mm×867 mm positive electrode plate.

**Examples 2-1 to 2-13**

These examples were the same as Example 1-4 except that in <Preparation of electrolyte>, the phosphonitrile compounds were further added according to the types and percentages shown in Table 2.

**Examples 3-1 to 3-11**

These examples were the same as Example 1-4 except that in <Preparation of electrolyte>, the linear carboxylates were further added according to the types and percentages shown in Table 3.

**Examples 3-12 and 3-13**

These examples were the same as Example 1-4 except that in <Preparation of electrolyte>, the linear carboxylates were further added according to the types and percentages shown in Table 3, and the positive electrode plates were prepared according to the following method.

<Preparation of positive electrode plate>

A positive electrode material $LiCoO_2$, a conductive agent conductive carbon black, and a binder polyvinylidene difluoride were mixed at a mass ratio of 95:2:3, N-methylpyrrolidone (NMP) was added, and the mixture was stirred to uniformity using a vacuum stirrer to produce a positive electrode slurry, where a solid content of the positive electrode slurry was 70wt%. Aluminum oxide and the binder PVDF at a mass ratio of 90:10 were mixed with the NMP to produce an insulating layer slurry. The positive electrode slurry and the insulating layer slurry were uniformly applied onto one surface of a 12 μm thick positive electrode current collector aluminum foil, and the aluminum foil was dried at 85°C for 4 h to obtain a positive electrode plate sequentially coated with a 130 μm thick and 74 mm wide positive electrode active material layer and a 130 μm thick and 2 mm wide insulating layer on one surface. The same steps were repeated on another surface of the aluminum foil to obtain a positive electrode plate sequentially coated with positive electrode active material layers and insulating layers on both surfaces. Then, the positive electrode plate was dried in vacuum at 85°C for 4 h, followed by cold pressing, cutting, and slitting, to obtain a 78 mm×867 mm positive electrode plate.

**Examples 4-1 to 4-9**

These examples were the same as Example 2-3 except that in <Preparation of electrolyte>, the linear carboxylates were further added according to the types and percentages shown in Table 4.

**Comparative Examples 1 to 7**

These comparative examples were the same as Example 1-1 except that the related preparation parameters were adjusted according to Table 1.

Preparation parameters and performance parameters of the examples and comparative examples are shown in Tables 1 to 4.

**Table 1**

| | Polynitrile compound | Percentage N of polynitrile compound (%) | M (mm) | M×N | Negative electrode impedance increase rate (%) | Floating charge thickness swelling rate (%) |
|---|---|---|---|---|---|---|
| Example 1-1 | Formula (I-18) | 0.01 | 1.3 | 0.013 | 145 | 20.5 |
| Example 1-2 | Formula (I-18) | 0.5 | 1.3 | 0.65 | 140 | 17.8 |
| Example 1-3 | Formula (I-18) | 1 | 1.3 | 1.3 | 138 | 16.2 |
| Example 1-4 | Formula (I-18) | 2 | 1.3 | 2.6 | 123 | 12.5 |
| Example 1-5 | Formula (I-18) | 3 | 1.3 | 3.9 | 127 | 12.8 |
| Example 1-6 | Formula (I-18) | 5 | 1.3 | 6.5 | 137 | 16.6 |
| Example 1-7 | Formula (I-18) | 8 | 1.2 | 9.6 | 143 | 21.3 |

(continued)

| | Polynitrile compound | Percentage N of polynitrile compound (%) | M (mm) | M×N | Negative electrode impedance increase rate (%) | Floating charge thickness swelling rate (%) |
|---|---|---|---|---|---|---|
| Example 1-8 | Formula (I-18) | 1 | 0.5 | 0.5 | 148 | 22.9 |
| Example 1-9 | Formula (I-18) | 1 | 2 | 2 | 142 | 20.6 |
| Example 1-10 | 1,3,6-hexanetricarbonitrile | 1 | 1.3 | 1.3 | 147 | 17.6 |
| Example 1-11 | Formula(I-19) | 1 | 1.3 | 1.3 | 142 | 16.9 |
| Example 1-12 | Formula (I-17) | 1 | 1.3 | 1.3 | 140 | 16.8 |
| Example 1-13 | Formula (I-8) | 1 | 1.3 | 1.3 | 145 | 17.1 |
| Example 1-14 | Formula (I-18)+Formula (I-8) | 0.5+0.5 | 1.3 | 1.3 | 135 | 15.8 |
| Example 1-15 | Formula (I-18) | 1 | 1.3 | 1.3 | 136 | 15.6 |
| Comparative Example 1 | \ | \ | 1.3 | \ | 166 | 28.9 |
| Comparative Example 2 | Formula (I-18) | 10 | 1.3 | 13 | 159 | 25.7 |
| Comparative Example 3 | Formula (I-18) | 3 | 0.4 | 1.2 | 201 | 39.8 |
| Comparative Example 4 | Formula (I-18) | 3 | 2.5 | 7.5 | 174 | 29.8 |
| Comparative Example 5 | \ | \ | 2.5 | \ | 168 | 29.0 |
| Comparative Example 6 | Adiponitrile | 3 | 1.3 | 3.9 | 149 | 22.8 |
| Comparative Example 7 | Succinonitrile | 3 | 1.3 | 3.9 | 145 | 21.8 |
| Note: "\" in Table 1 means that a related preparation parameter does not exist. In Examples 1-7 to 1-9 and Comparative Examples 3 to 5, when M changes, the width of the negative electrode plate changes accordingly, while the width of the positive electrode plate remains unchanged. | | | | | | |

It can be learned from Examples 1-1 to 1-14 and Comparative Examples 1 to 7 that the negative electrode impedance increase rate and floating charge thickness swelling rate of the lithium-ion battery vary with the type and mass percentage N% of the polynitrile compound, the width M of the protruding region, and the value of M×N. The lithium-ion battery, with the type and mass percentage N% of the polynitrile compound, the width M of the protruding region, and the value of M×N within the ranges of this application, has smaller negative electrode impedance increase rate and floating charge thickness swelling rate, indicating better floating charge performance of the lithium-ion battery.

It can be learned from comparison between Examples 1-1 to 1-7 and Comparative Example 2 that when the mass percentage N% of the polynitrile compound in the electrolyte is greater than 8%, the negative electrode impedance and floating charge performance of the electrochemical device are both affected. It can be learned from comparison between Example 1-5 and Comparative Examples 3 and 4 that when the width M mm of the protruding region is less than 0.5 mm or greater than 2 mm, the negative electrode impedance and floating charge performance of the electrochemical device are both affected. It can be learned from comparison between Examples 1-3 and 1-11 to 1-13 and Example 1-10 that when the polynitrile compound includes a carbon-oxygen single bond structure, the electrochemical device has further reduced negative electrode impedance and improved floating charge performance. It can be learned from comparison between

Example 1-5 and Comparative Examples 6 and 7 that when the polynitrile compound in this application is replaced with another nitrile compound, the negative electrode impedance increase rate and the floating charge swelling rate cannot be significantly reduced.

**Table 2**

| | Polynitrile compound | N | Phosphonitrile compound | P | N+P (%) | Negative electrode impedance increase rate (%) | Floating charge thickness swelling rate (%) | Hot-box test (pass number/test number) |
|---|---|---|---|---|---|---|---|---|
| Example 1-4 | Formula (I-18) | 2 | \ | \ | 2 | 123 | 12.5 | 5/10 |
| Example 2-1 | Formula (I-18) | 2 | Formula (II-2) | 0.01 | 2.01 | 119 | 11.8 | 6/10 |
| Example 2-2 | Formula (I-18) | 2 | Formula (II-2) | 0.5 | 2.5 | 117 | 11.3 | 8/10 |
| Example 2-3 | Formula (I-18) | 2 | Formula (II-2) | 1 | 3 | 112 | 10.8 | 10/10 |
| Example 2-4 | Formula (I-18) | 2 | Formula (II-2) | 2 | 4 | 118 | 11.4 | 10/10 |
| Example 2-5 | Formula (I-18) | 2 | Formula (II-2) | 4.5 | 6.5 | 119 | 11.5 | 10/10 |
| Example 2-6 | Formula (I-18) | 2 | Formula (II-2) | 6 | 8 | 121 | 11.9 | 8/10 |
| Example 2-7 | Formula (I-18) | 0.01 | Formula (II-2) | 0.09 | 0.1 | 125 | 12.8 | 7/10 |
| Example 2-8 | Formula (I-18) | 5 | Formula (II-2) | 5 | 10 | 128 | 13.2 | 8/10 |
| Example 2-9 | Formula (I-18) | 2 | Formula (II-1) | 1 | 3 | 117 | 11.9 | 9/10 |
| Example 2-10 | Formula (I-18) | 2 | Formula (II-5) | 1 | 3 | 114 | 11.6 | 10/10 |
| Example 2-11 | Formula (I-18) | 2 | Formula (II-6) | 1 | 3 | 117 | 11.3 | 8/10 |
| Example 2-12 | Formula (I-18) | 2 | Formula (II-19) | 1 | 3 | 116 | 11.6 | 9/10 |
| Example 2-13 | Formula (I-18) | 2 | Formula (II-2) +Formula (II-5) | 1 | 3 | 113 | 11.2 | 10/10 |
| Note: "\" in Table 2 means that a related preparation parameter does not exist. | | | | | | | | |

With the phosphonitrile compound further added to the electrolyte, the lithium-ion battery has both good floating charge performance and good safety performance. The mass percentage P% of the phosphonitrile compound generally also affects the negative electrode impedance increase rate, floating charge thickness swelling rate, and hot-box test of the lithium-ion battery. It can be learned from Examples 1-4 and 2-1 to 2-6 that the lithium-ion battery, with the phosphonitrile compound added to the electrolyte and the mass percentage P% of the phosphonitrile compound within the range of this application, has both lower negative electrode impedance increase rate and floating charge thickness swelling rate and higher pass number of hot-box test, indicating good floating charge performance and safety performance of the lithium-ion battery.

The value of N+P generally also affects the negative electrode impedance increase rate, floating charge thickness swelling rate, and hot-box test of the lithium-ion battery. It can be learned from Examples 2-1 to 2-8 that the lithium-ion battery, with the value of N+P within the range of this application, has lower negative electrode impedance

increase rate and floating charge thickness swelling rate and higher pass number of hot-box test, indicating good floating charge performance and safety performance of the lithium-ion battery.

The type of the phosphonitrile compound generally also affects the negative electrode impedance increase rate, floating charge thickness swelling rate, and hot-box test of the lithium-ion battery. It can be learned from Examples 2-3 and 2-9 to 2-13 that the lithium-ion battery, with the type of the phosphonitrile compound within the range of this application, has lower negative electrode impedance increase rate and floating charge thickness swelling rate and higher pass number of hot-box test, indicating good floating charge performance and safety performance of the lithium-ion battery.

**Table 3**

| | Polynitrile compound | N | Linear carboxylate | X | N/X | Negative electrode impedance increase rate (%) | Floating charge thickness swelling rate (%) |
|---|---|---|---|---|---|---|---|
| Example 1-4 | Formula (I-18) | 2 | \ | \ | 2 | 123 | 12.5 |
| Example 3-1 | Formula (I-18) | 2 | Propyl propionate | 10 | 0.2 | 112 | 10.4 |
| Example 3-2 | Formula (I-18) | 2 | Ethyl propionate | 10 | 0.2 | 114 | 10.6 |
| Example 3-3 | Formula (I-18) | 2 | Ethyl acetate | 10 | 0.2 | 115 | 10.8 |
| Example 3-4 | Formula (I-18) | 2 | Propyl acetate | 10 | 0.2 | 117 | 11.2 |
| Example 3-5 | Formula (I-18) | 2 | Propyl propionate | 5 | 0.4 | 119 | 11.8 |
| Example 3-6 | Formula (I-18) | 2 | Propyl propionate | 8 | 0.25 | 116 | 11.1 |
| Example 3-7 | Formula (I-18) | 2 | Propyl propionate | 15 | 0.133 | 115 | 10.6 |
| Example 3-8 | Formula (I-18) | 2 | Propyl propionate | 20 | 0.1 | 115 | 10.9 |
| Example 3-9 | Formula (I-18) | 2 | Propyl propionate | 40 | 0.05 | 118 | 11.6 |
| Example 3-10 | Formula (I-18) | 2 | Propyl propionate | 60 | 0.033 | 121 | 12.1 |
| Example 3-11 | Formula (I-18) | 0.5 | Propyl propionate | 50 | 0.01 | 123 | 12.4 |
| Example 3-12 | Formula (I-18) | 2 | Propyl propionate | 20 | 0.1 | 107 | 9.3 |
| Example 3-13 | Formula (I-18) | 2 | Ethyl propionate | 20 | 0.1 | 109 | 9.4 |
| Note: "\" in Table 3 means that a related preparation parameter does not exist. | | | | | | | |

With the linear carboxylate further added to the electrolyte, the floating charge performance of the lithium-ion battery can be further improved. The mass percentage X% of the linear carboxylate generally also affects the negative electrode impedance increase rate and floating charge thickness swelling rate of the lithium-ion battery. It can be learned from Examples 1-4, 3-1, and 3-5 to 3-11 that the lithium-ion battery, with the linear carboxylate added to the electrolyte and the mass percentage X% of the linear carboxylate within the range of this application, has lower negative electrode impedance increase rate and lower floating charge thickness swelling rate, indicating better floating charge performance of the lithium-ion battery.

The type of the linear carboxylate generally also affects the negative electrode impedance increase rate and floating charge thickness swelling rate of the lithium-ion battery. It can be learned from Examples 3-1 to 3-4 that the lithium-ion battery, with the type of the linear carboxylate within the range of this application, has lower negative electrode impedance increase rate and floating charge thickness swelling rate, indicating good floating charge performance of the lithium-ion battery.

The value of N/X generally also affects the negative electrode impedance increase rate and floating charge thickness swelling rate of the lithium-ion battery. It can be learned from Examples 3-1 and 3-5 to 3-11 that the lithium-ion battery, with the value of N/X within the range of this application, has lower negative electrode impedance increase rate and floating charge thickness swelling rate, indicating good floating charge performance of the lithium-ion battery.

**Table 4**

| | Polynitrile compound | N | Phosphonitrile compound | P | Linear carboxylate | X | X/ (5N+P) | Negative electrode impedance increase rate (%) | Floating charge thickness swelling rate (%) | Hot-box test (pass number/test number) |
|---|---|---|---|---|---|---|---|---|---|---|
| Example 1-4 | Formula (I-18) | 2 | \ | \ | \ | \ | \ | 123 | 12.5 | 5/10 |
| Example 2-3 | Formula (I-18) | 2 | Formula (II-2) | 1 | \ | \ | \ | 112 | 10.8 | 10/10 |
| Example 4-1 | Formula (I-18) | 2 | Formula (II-2) | 2 | Propyl propionate | 10 | 0.91 | 108 | 9.2 | 10/10 |
| Example 4-5 | Formula (I-18) | 2 | Formula (II-2) | 2 | Propyl propionate | 5 | 0.45 | 110 | 10.4 | 10/10 |
| Example 4-6 | Formula (I-18) | 2 | Formula (II-2) | 2 | Propyl propionate | 20 | 1.82 | 105 | 8.9 | 10/10 |
| Example 4-7 | Formula (I-18) | 2 | Formula (II-2) | 2 | Propyl propionate | 40 | 3.64 | 110 | 9.8 | 10/10 |
| Example 4-8 | Formula (I-18) | 2 | Formula (II-2) | 2 | Propyl propionate | 60 | 5.45 | 110 | 10.3 | 10/10 |
| Example 4-9 | Formula (I-18) | 0.5 | Formula (II-2) | 0.5 | Propyl propionate | 60 | 20 | 111 | 10.6 | 10/10 |
| Note: "\" in Table 4 means that a related preparation parameter does not exist. | | | | | | | | | | |

With all of the polynitrile compound, the phosphonitrile compound, and the linear carboxylate added to the electrolyte, the value of the relation X/(5N+P) of the mass percentage N% of the polynitrile compound, the mass percentage P% of the phosphonitrile compound, and the mass percentage X% of the linear carboxylate generally also affects the negative electrode impedance increase rate, floating charge thickness swelling rate, and hot-box test of the lithium-ion battery. It can be learned from Examples 1-4, 2-3, and 4-1 to 4-9 that the lithium-ion battery, with the value of X/(5N+P) within the range of this application, has lower negative electrode impedance increase rate and floating charge thickness swelling rate, and most of such batteries have higher pass number of hot-box test, indicating good floating charge performance and safety performance of the lithium-ion battery.

The foregoing descriptions are merely preferred embodiments of this application, and are not intended to limit this application. Any modifications, equivalent replacements, improvements, and the like made without departing from the spirit and principle of this application shall fall within the protection scope of this application.

## Claims

**1.** An electrochemical device, comprising a positive electrode plate, a separator, and a negative electrode plate, wherein

the positive electrode plate comprises a positive electrode active material layer, and the negative electrode plate comprises a negative electrode active material layer, and
observed along a thickness direction of the electrochemical device, in a width direction of the negative electrode plate, the negative electrode active material layer comprises two protruding regions not overlapping with the positive electrode active material layer on two sides, and a width of the protruding region is M mm, wherein $0.5 \leq M \leq 2$; and
the electrochemical device further comprises an electrolyte, the electrolyte comprises a polynitrile compound, and the polynitrile compound comprises a compound represented by formula I-A:

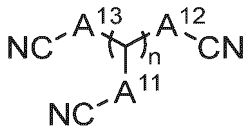
formula I-A;

wherein
in formula I-A, $A^{11}$, $A^{12}$, and $A^{13}$ are independently selected from one of formula (I-A1) or formula (I-A2):

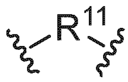
formula (I-A1)

or 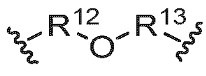 formula (I-A2);

and
in formula I-A, n is a positive integer from 1 to 8, wherein
when multiple $A^{11}$ are present, the multiple $A^{11}$ may be the same or different;
$R^{11}$, $R^{12}$, and $R^{13}$ are independently selected from a covalent single bond, substituted or unsubstituted $C_1$-$C_{10}$ alkylene group, substituted or unsubstituted $C_2$-$C_{10}$ alkenylene group, substituted or unsubstituted $C_2$-$C_{10}$ alkynylene group, substituted or unsubstituted $C_6$-$C_{10}$ arylene group, substituted or unsubstituted $C_3$-$C_{10}$ cycloalkylene group, or substituted or unsubstituted $C_1$-$C_{10}$ heterocyclene group, wherein when the foregoing groups are substituted, a substituent group is selected from halogen; and
the heterocyclene group comprises heteroalicyclene group and heterocycloarylene group, and heteroatom in the heterocyclene group is selected from at least one of O, N, S, or Si; wherein

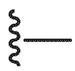

represents a bonding site with an adjacent atom;
based on a mass of the electrolyte, a mass percentage N% of the polynitrile compound is 0.01% to 8%; and the width M of the protruding region and the mass percentage N% of the polynitrile compound satisfy $0.01 \leq M \times N < 10$.

2. The electrochemical device according to claim 1, wherein at least two of $A^{11}$, $A^{12}$, and $A^{13}$ are selected from formula (I-A2).

3. The electrochemical device according to claim 1, wherein the width M of the protruding region and the mass percentage N% of the polynitrile compound satisfy $0.5 < M \times N \leq 7$, and preferably $2.6 \leq M \times N \leq 3.9$.

4. The electrochemical device according to claim 1, wherein the compound represented by formula I-A comprises at least one of compounds represented by formulas (I-1) to (I-20):

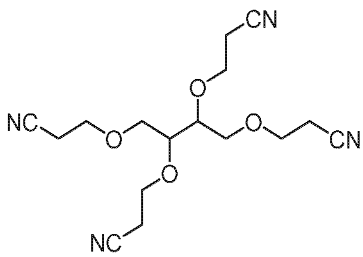
formula (I-1),

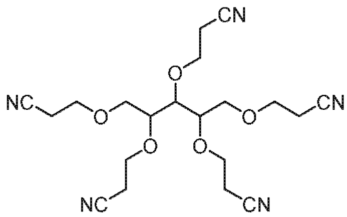

formula (I-2),

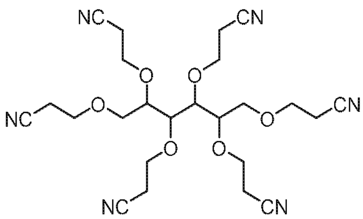

formula (I-3),

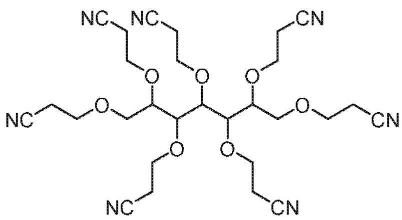

formula (I-4),

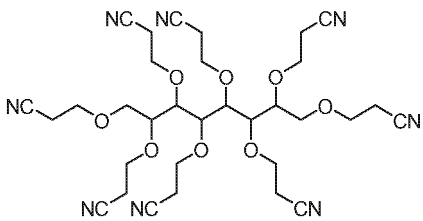

formula (I-5),

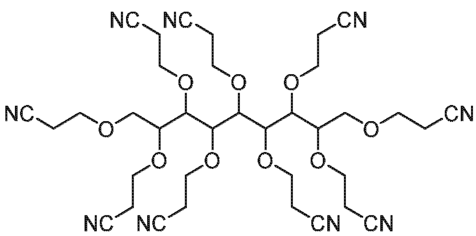

formula (I-6),

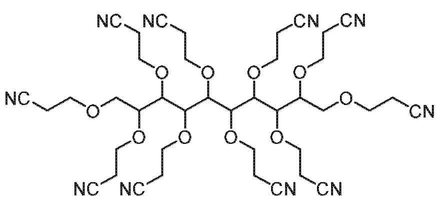

formula (I-7),

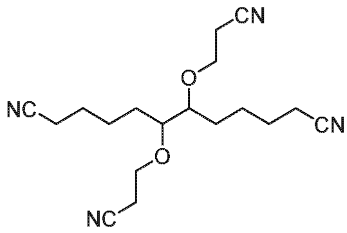

formula (I-8),

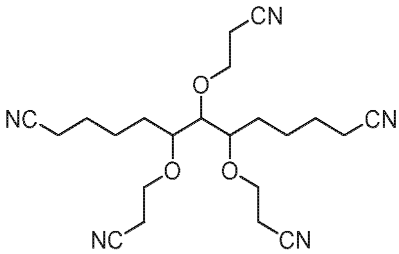

formula (I-9),

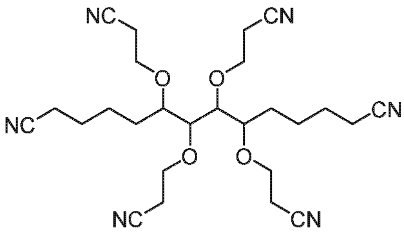

formula (I-10),

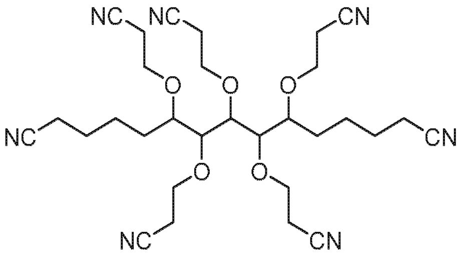

formula (I-11),

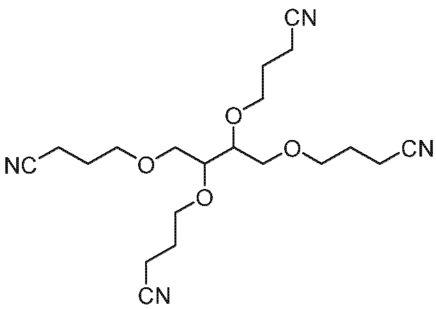

formula (I-12),

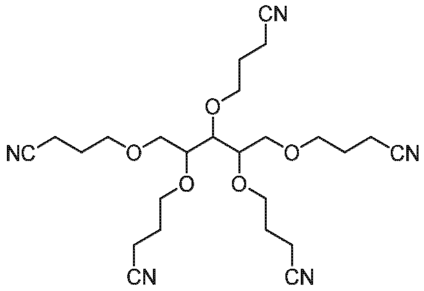
formula (I-13),

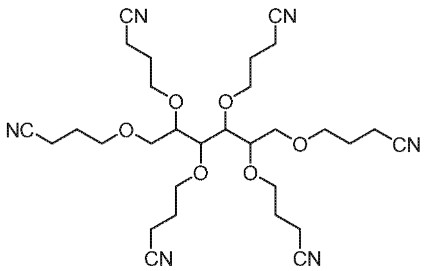
formula (I-14),

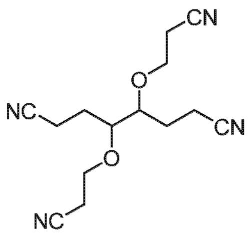
formula (I-15),

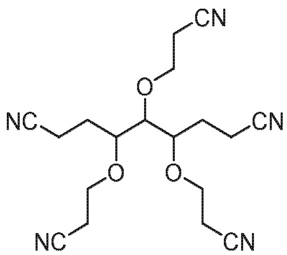
formula (I-16),

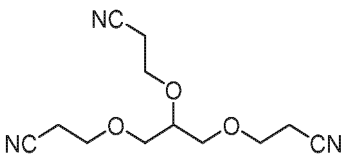
formula (I-17),

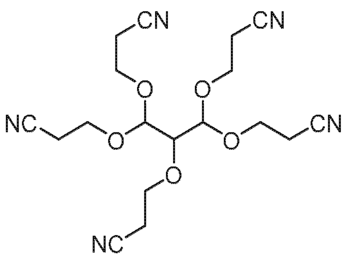
formula (I-18),

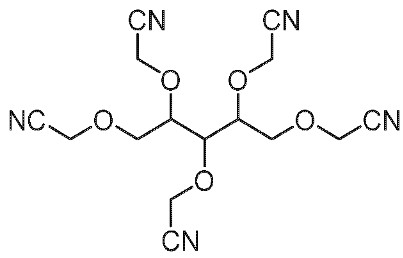

formula (I-19), or

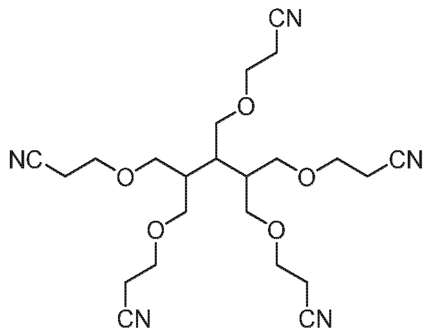

formula (I-20).

5. The electrochemical device according to claim 1, wherein the compound represented by formula I-A comprises at least one of 1,2,3-tris(2-cyanoethoxy)propane or 1,3,6-hexanetricarbonitrile.

6. The electrochemical device according to claim 1, wherein the electrolyte further comprises a phosphonitrile compound, and the phosphonitrile compound comprises a compound represented by formula II-A:

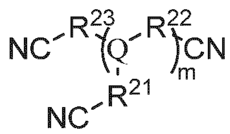

formula II-A;

wherein

in formula II-A, Q is independently selected from formula (II-A1) or formula (II-A2):

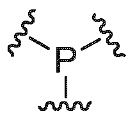

formula (II-A1) or

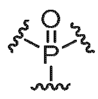

formula (II-A2);

in formula II-A, m is selected from 1 or 2, each Q may be the same or different, and each $R^{22}$ may be the same or different;

$R^{21}$, $R^{22}$, and $R^{23}$ are independently selected from a covalent single bond, substituted or unsubstituted $C_1$-$C_{10}$ alkylene group, substituted or unsubstituted $C_2$-$C_{10}$ alkenylene group, substituted or unsubstituted $C_2$-$C_{10}$ alkynylene group, substituted or unsubstituted $C_6$-$C_{10}$ arylene group, substituted or unsubstituted $C_3$-$C_{10}$ cycloalkylene group, or substituted or unsubstituted $C_1$-$C_{10}$ heterocyclene group, wherein when the foregoing groups are substituted, a substituent group is selected from halogen; and

the heterocyclene group comprises heteroalicyclene group and heterocycloarylene group, and heteroatom in the heterocyclene group is selected from at least one of O, N, S, or Si; wherein

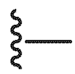

represents a bonding site with an adjacent atom; and

based on the mass of the electrolyte, a mass percentage P% of the phosphonitrile compound is 0.01% to 6%.

7. The electrochemical device according to claim 1, wherein the compound represented by formula II -A comprises at least one of compounds represented by formulas (II -1) to (II -21):

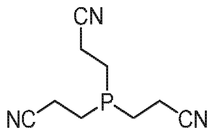
formula (II-1),

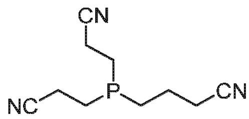
formula (II-2),

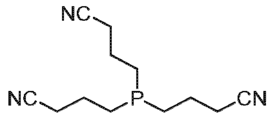
formula (II-3),

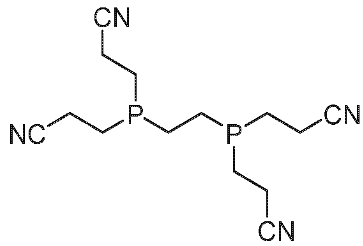
formula (II-4),

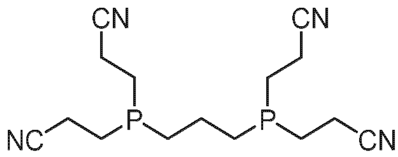
formula (II-5),

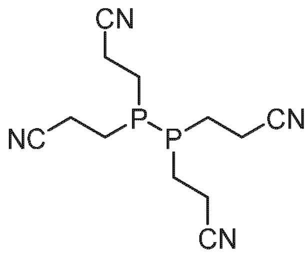
formula (II-6),

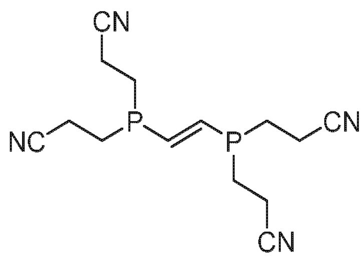
formula (II-7),

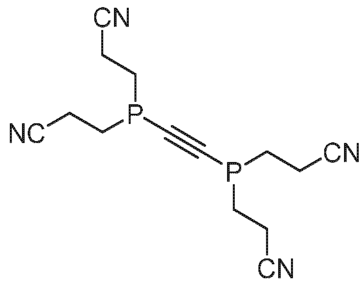
formula (II-8),

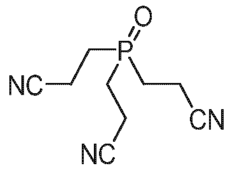
formula (II-9),

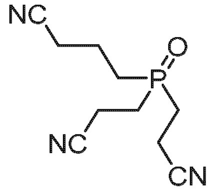
formula (II-10),

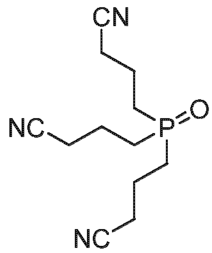
formula (II-11),

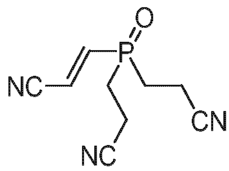
formula (II-12),

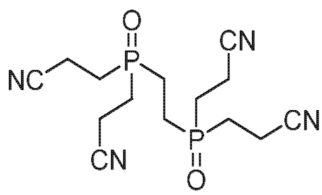
formula (II-13),

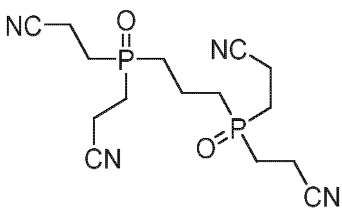
formula (II-14),

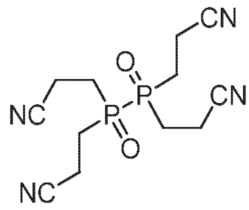

formula (II-15),

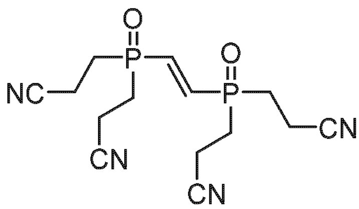

formula (II-16),

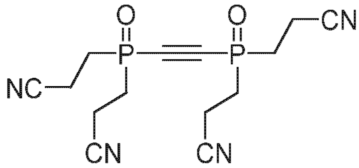

formula (II-17),

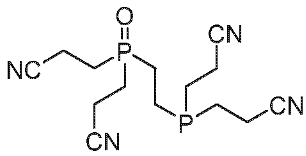

formula (II-18),

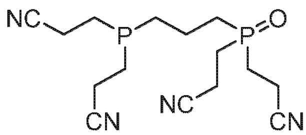

formula (II-19),

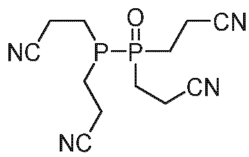

formula (II-20), or

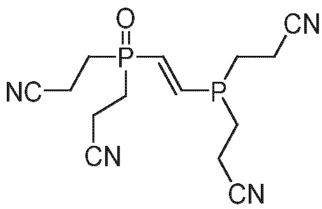

formula (II-21).

**8.** The electrochemical device according to claim 6, wherein the mass percentage N% of the polynitrile compound and the mass percentage P% of the phosphonitrile compound satisfy 0.1 ≤ N+P ≤ 10, and preferably 2 ≤ N+P ≤ 8.

**9.** The electrochemical device according to any one of claims 1 to 6, wherein the electrolyte further comprises linear carboxylate, and based on the mass of the electrolyte, a mass percentage X% of the linear carboxylate is 1% to 60%.

**10.** The electrochemical device according to claim 9, wherein the linear carboxylate comprises at least one of ethyl

acetate, propyl acetate, butyl acetate, ethyl propionate, propyl propionate, butyl butyrate, methyl butyrate, methyl isovalerate, propyl hexanoate, or isobutyl acetate.

**11.** The electrochemical device according to claim 9, wherein the mass percentage N% of the polynitrile compound and the mass percentage X% of the linear carboxylate satisfy $N/X \geq 0.01$, and preferably $0.05 \leq N/X \leq 0.4$.

**12.** The electrochemical device according to claim 1, wherein the electrolyte further comprises a phosphonitrile compound and linear carboxylate; wherein
based on the mass of the electrolyte, the mass percentage N% of the polynitrile compound, a mass percentage P% of the phosphonitrile compound, and a mass percentage X% of the linear carboxylate satisfy $X/(5N+P) \leq 20$, and preferably $0.4 \leq X/(5N+P) \leq 10$.

**13.** The electrochemical device according to claim 1, wherein the positive electrode plate comprises a positive electrode current collector, an insulating layer is provided on a surface of the positive electrode current collector, and in a width direction of the positive electrode plate, the insulating layer is disposed on two sides of the positive electrode active material layer, wherein a width of the insulating layer is 1.5 mm to 5 mm.

**14.** The electrochemical device according to any one of claims 1 to 13, wherein the electrochemical device satisfies at least one of the following:

(a) $2.6 \leq M \times N \leq 3.9$;
(b) $2.5 \leq N+P \leq 6.5$;
(c) $0.1 \leq N/X \leq 0.25$; or
(d) $0.91 \leq X/(5N+P) \leq 3.64$.

**15.** An electronic device, comprising the electrochemical device according to any one of claims 1 to 14.

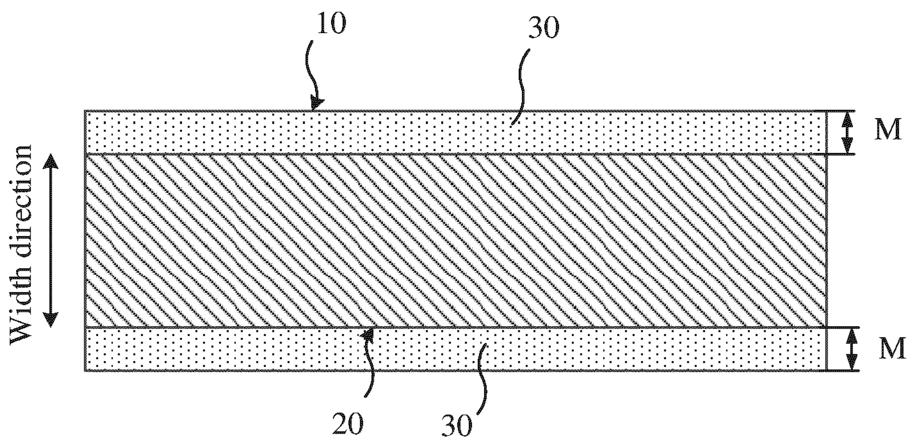


FIG. 1

**INTERNATIONAL SEARCH REPORT**

International application No. **PCT/CN2022/105202**

**A. CLASSIFICATION OF SUBJECT MATTER**

H01M10/0567(2010.01)i;H01M10/0525(2010.01)i

According to International Patent Classification (IPC) or to both national classification and IPC

**B. FIELDS SEARCHED**

Minimum documentation searched (classification system followed by classification symbols)

IPC：H01M

Documentation searched other than minimum documentation to the extent that such documents are included in the fields searched

Electronic data base consulted during the international search (name of data base and, where practicable, search terms used)

CNABS; CNTXT; CNKI; VEN; USTXT; EPTXT; WOTXT: 负极, 伸出, 宽度, 腈, 氰, extend+, width, hang, over, +nitril+, negative electrode, HTCN

**C. DOCUMENTS CONSIDERED TO BE RELEVANT**

| Category* | Citation of document, with indication, where appropriate, of the relevant passages | Relevant to claim No. |
|---|---|---|
| X | JP 2018006046 A (SAMSUNG SDI CO., LTD.) 11 January 2018 (2018-01-11) description, paragraphs [0009]-[0117], and figure 1 | 1-15 |
| X | CN 105576279 A (HITACHI MAXELL) 11 May 2016 (2016-05-11) description, paragraphs [0014]-[0246], and figures 1 and 2 | 1-15 |
| A | CN 113707867 A (NINGDE AMPEREX TECHNOLOGY LTD.) 26 November 2021 (2021-11-26) entire document | 1-15 |

☐ Further documents are listed in the continuation of Box C.  ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **9.2月2023（09.02.2023）** | **09 March 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| Facsimile No. **(86-10)62019451** | Telephone No. |

Form PCT/ISA/210 (second sheet) (July 2022)

**INTERNATIONAL SEARCH REPORT**
**Information on patent family members**

International application No.
**PCT/CN2022/105202**

| Patent document cited in search report | Publication date (day/month/year) | Patent family member(s) | Publication date (day/month/year) |
|---|---|---|---|
| JP 2018006046 A | 11 January 2018 | KR 20180001989 A | 05 January 2018 |
| | | KR 102215327 B1 | 10 February 2021 |
| | | JP 6787700 B2 | 18 November 2020 |
| CN 105576279 A | 11 May 2016 | KR 20160052382 A | 12 May 2016 |
| | | KR 102230042 B1 | 19 March 2021 |
| | | JP 2016085949 A | 19 May 2016 |
| | | JP 6453611 B2 | 16 January 2019 |
| | | CN 105576279 B | 12 May 2020 |
| CN 113707867 A | 26 November 2021 | None | |

Form PCT/ISA/210 (patent family annex) (July 2022)